# EUROPÄISCHE PATENTSCHRIFT

(11) **EP 1 146 853 B1**
(45) Veröffentlichungstag und Bekanntmachung des Hinweises auf die Patenterteilung: **02.04.2003**
(21) Anmeldenummer: 00910599.0
(22) Anmeldetag: 21.01.2000
(51) Int. Cl.: A61K 7/48

(54) **ANTIOXIDATIVE HAUTPFLEGEMITTEL**
ANTIOXIDANT SKIN CARE PRODUCTS
AGENT DE SOIN ANTIOXYDANT POUR LA PEAU

(30) Priorität: 30.01.1999 DE 19903716
(43) Veröffentlichungstag der Anmeldung: 24.10.2001
(73) Patentinhaber: Henkel Kommanditgesellschaft auf Aktien, 40589 Düsseldorf (DE)
(72) Erfinder: MÜLLER, Angela, D-41515 Grevenbroich (DE); WALDMANN-LAUE, Marianne, D-40789 Monheim (DE); HAMMES, Christina, D-51061 Köln (DE); ORTANDERL, Stefanie, D-53894 Mechernich (DE); BLUMENKAMP, Elke, D-41751 Viersen (DE); MUNK, Gabriele, D-22763 Hamburg (DE)
(86) Internationale Anmeldenummer: EP0000451
(87) Internationale Veröffentlichungsnummer: WO00044344

(56) Entgegenhaltungen:
- EP-A- 0 440 398
- EP-A- 0 659 402
- WO-A-99/30682
- WO-A-99/66881
- DE-A- 19 827 624
- US-A- 4 673 530
- US-A- 5 972 993
- DATABASE WPI Week 199625 Derwent Publications Ltd., London, GB; AN 1996-246866 XP002144762 & JP 08 099827 A (SHISEIDO CO)
- PATENT ABSTRACTS OF JAPAN vol. 014, no. 290 (C-0731) & JP 02 092258 A (SANKYO CO)

## Beschreibung

Die Erfindung betrifft kosmetische Zubereitungen mit einer synergistisch wirkenden Antioxidantien-Kombination, die als Hautpflegemittel Anwendung finden.

Als äußeres Organ mit der größten Oberfläche ist die Haut Umwelteinflüssen in besonderem Maße ausgesetzt. Da die Haut vornehmlich dem Schutz des Körpers dient, verfügt sie über zahlreiche natürliche Schutzbarrieren und Regenerationsprozesse. Zunehmende Schadstoff- und UV-Belastung in jüngster Zeit führen jedoch zu einer übermäßigen Strapazierung der Haut und stören das natürliche Regenerationsvermögen auf empfindliche Weise. So führt eine übermäßige Belastung durch energiereiche UV-Strahlen sowohl zu akuten Schäden, wie z. B. Sonnenbrand, als auch zu chronischen Veränderungen, wie beispielsweise vorzeitiger Hautalterung in Form von strukturellen Veränderungen des Bindegewebes. Als Folge der übermäßigen UV-Belastung bilden sich in der Haut verschiedene reaktive Sauerstoff-Spezies, die auslösend oder verstärkend auf zahlreiche unerwünschte oxidative Prozesse wirken. Um den sogenannten "oxidativen Stress" zu kontrollieren, enthält die Haut verschiedene antioxidativ wirkende Substanzen, z. B. Superoxid-Dismutase, Tocopherole und Ascorbinsäure. Diese Antioxidantien verhindern, daß freie Sauerstoff-Radikale die Lipid-Membranen der lebenden Zellen angreifen (Lipidperoxidation) oder gar zu irreversiblen Schäden des Erbmaterials führen.
Neuere Forschungen haben gezeigt, daß auch die externe Anwendung von Antioxidantien den oxidativen Stress vermindern kann. Die Bemühungen der Kosmetik-Industrie haben die Entwicklung von Körperpflegemitteln gefördert, die als Präventivmaßnahme hochwirksame und insbesondere physiologisch unbedenkliche Antioxidantien enthalten. In jüngster Zeit werden vor allem synergistisch wirkende Mehr-Komponenten-Systeme eingesetzt, wobei vermehrt Naturstoff-Extrakte zum Einsatz kommen.

Dem Fachmann sind zahlreiche pflanzliche Extrakte mit antioxidativen Wirkstoffen bekannt (H. Eggensperger, Pflanzliche Wirkstoffe für Kosmetika, Melcher Verlag GmbH, München, 1995, S. 402 ff.) Ein Verfahren zur Gewinnung einer natürlichen Antioxidantien-Mischung aus grünem Tee wird in US 4673530 gelehrt. Ein Hautpflegemittel auf Basis von α-Tocopherylretinoat wird in JP 8099821 beschrieben. Der synergistische oder kooperative antioxidative Effekt einer Mischung aus Procyanidinen und Vitamin E wurde von M. Caribi et al. beschrieben [*International Journal of Cosmetic Science* **20,** 203-215, (1998)].

Die derzeit eingesetzten Antioxidantien oder Antioxidans-Mischungen können den Anforderungen jedoch noch nicht völlig gerecht werden. Naturstoffextrakte mit synergistisch wirkenden Antioxidantien führen häufig zu einer unerwünschten Verfärbung der Kosmetika und können daher nur bedingt verwendet werden.

Aufgabe war es daher, eine Antioxidantien-Kombination zu entwickeln, die eine hohe synergistische Wirkung entfaltet ("Booster-Effekt") und mit den Komponenten des Hautpflegemittels kompatibel ist. Unter einer synergistischen Wirkung im Sinne der Erfindung wird ein kooperativer Effekt der Antioxidantien verstanden, d.h. daß die antioxidative Wirkung bei Kombination der Einzelkomponenten nicht additiv ist, sondern um ein Vielfaches verstärkt wird. Die antioxidative Wirkung oder das sogenannte antioxidative Potential wurde mittels einer Chemilumineszenz-Methode in Analogie zu DD 249 618 A3 erfaßt.

Überraschenderweise wurde nun eine Antioxidantien-Kombination mit einem hohen synergistischen Effekt gefunden, die mit den physiologischen Trägern und anderen Inhaltsstoffen eines Hautpflegemittels kompatibel ist und sich geruchs- und farbneutral verhält. Zubereitungen, die diese Kombination von Antioxidantien enthalten, haben nicht nur ausgezeichnete pflegende Eigenschaften, sondern können der Hautalterung durch oxidative Prozesse vorbeugen und/oder das Erscheinungsbild der Altershaut verbessern.

Gegenstand der Erfindung ist daher eine kosmetische Zubereitung, dadurch gekennzeichnet, daß als Antioxidans eine Kombination aus (a) mindestens einem Tocopherol und/oder Tocopherol-Ester, (b) mindestens einem Gallussäure-Ester und (c) einem Auszug der Tee-Pflanze in einem zur topischen Applikation auf der Haut geeigneten Träger enthalten ist.

Die Antioxidantien-Mischung enthält Tocopherole und/oder Tocopherol-Ester als obligatorische Komponente (a). Als Antioxidantien oder Oxidationsinhibitoren werden organische Verbindungen unterschiedlicher Verbindungsklassen eingesetzt, die durch Sauerstoff-Einwirkung oder andere oxidative Prozesse bedingte Veränderungen teilweise oder vollständig inhibieren. Wegen ihrer physiologischen Unbedenklichkeit und ihrer leichten Zugänglichkeit finden Tocopherole und deren Ester vielseitige Anwendung als Antioxidantien in Kosmetika sowie in Lebensmitteln. Tocopherole, schwach gelblich bis rötliche Flüssigkeiten, sind 3,4-Dihydro-2*H*-1-benzopyran-6-ole, also Chroman-6-ole, die in 2-Stellung mit einem 4,8,12-Trimethyltridecyl-Rest substituiert sind. Sie kommen in vielen Pflanzenölen vor, insbesondere in Samenölen von Soja, Weizen, Mais, Reis, Baumwolle, Luzerne und Nüssen, aber auch in Früchten und Gemüsen. Als Tocopherole im Sinne der Erfindung eignen sich natürliche oder synthetisch zugängliche Tocopherole in enantiomerenreiner Form bzw. als Gemisch der Stereoisomeren, unter anderem α-, β-, γ-, δ-, und ε-Tocopherole. Eine bevorzugte Variante der erfindungsgemäßen Zubereitung ist dadurch gekennzeichnet, daß als Komponente (a) ein natürliches oder synthetisches α-Tocopherol in einer Menge von 0,005 - 2,0 Gew.-% der Gesamtzusammensetzung enthalten ist. Eine Menge von 0,1 - 1,0 Gew.-% gilt als besonders geeignet.

Als tocopherolhaltige Komponente ist insbesondere ein Produkt bevorzugt, das unter dem Handelsnamen Controx® KS bekannt ist. Alternativ zu oder in Kombination mit den Tocopherolen können Ester der Tocopherole eingesetzt werden. Als Ester im Sinne der Erfindung sind u. a. C₁-C₂₂ Alkyl- oder Arylester geeignet, aber auch Tocopherylsuccinat, Tocopherylpoly(oxyethylen)succinat, Tocopherylnicotinat und Tocopherylretinoat.

Eine weitere, bevorzugte Variante der erfindungsgemäßen Zubereitungen ist dadurch gekennzeichnet, daß neben α-Tocopherol zusätzlich α-Tocopherylacetat in einer Menge von 0,005 - 5,0 Gew.-% in der Zubereitung enthalten ist, wobei eine Menge von 0,1 - 2,0 Gew.-% als besonders bevorzugt gilt. Die Kombination der beiden Komponenten erweist sich insbesondere hinsichtlich der Farbneutralität der kosmetischen Zubereitung als relevant. Das Mengenverhältnis von Tocopherol : α-Tocopherylacetat beträgt üblicherweise 1: (2-10), wobei ein Verhältnis von 1: (2-5) als besonders bevorzugt gilt.

Die erfindungsgemäßen Zubereitungen enthalten weiterhin mindestens einen Gallussäure-Ester als obligatorische Komponente (b). Gallussäureester sind seit langem für ihre antioxidativen Eigenschaften bekannt. Zu den im Sinne der Erfindung geeigneten Estern zählen die Ester der Gallussäure (3,4,5-Trihydroxybenzoesäure) mit aliphatischen C₁-C₂₂-Alkholen. Zu den wichtigsten, in Kosmetika, Pharmazeutika und Lebensmitteln eingesetzten Vertretern gehören Methyl-, Ethyl-, Propyl-, Octyl-, Nonyl-, Dodecyl-, Cetyl-, und Stearylgallat. Eine bevorzugte Variante der erfindungsgemäßen Zubereitungen ist dadurch gekennzeichnet, daß als Gallussäureester Propylgallat in einer Menge von 0,005 - 2,0 Gew.-% der Gesamtzusammensetzung enthalten ist. Besonders geeignet ist ein Produkt, das unter dem Handelsnamen Danox® 200 bekannt ist.

Die dritte obligatorische Komponente (c) der Antioxidantien-Mischung ist ein Auszug der Tee-Pflanze (Camellia sinensis, Theaceae). Üblicherweise werden hierfür Blätter, Blattknospen und die zarten Stiele des Teestrauches verwendet, die als "Tee" im Handel sind. Im Sinne der Erfindung könnten ggf. jedoch auch andere Teile der Pflanze verwendet werden (Samen, Früchte, Blüten, Stengel, Rinden, Wurzeln). Man unterscheidet zwischen grünem, unfermentierten Tee und schwarzem, fermentierten Tee. Zu den Inhaltsstoffen des Tees zählen zahlreiche Polyphenolverbindungen, die für sein typisches Aroma verantwortlich sind. Neben Catechin- und Tannin-Gerbstoffen sowie Flavonoiden sind Coffein, Purin-Verbindungen, Proteine, Oligosaccharide, Stärke, Pektin, Inosit, Cellulose, Lignin, Oxalsäure, Fruchtsäuren und Spurenelemente enthalten. In den flüchtigen Bestandteilen des Tees wurden zwischen 300-400 Substanzen gefunden, von denen jedoch nur zwei identifiziert wurden. Die Zusammensetzung der Teeblätter variiert zudem beträchtlich nach Anbaugebiet, -läge und Sorte. Durch den Fermentationsprozeß wird der Gerbstoffgehalt in den Blättern stark reduziert. Die für Tee bekannte antioxidative Wirkung wird auf die darin enthaltenen Gerbstoffe zurückgeführt, insbesondere auf Epicatechin, Gallocatechin, Epigallocatechin, Epigallocatechingallat und Epicatechingallat.

Die Teeauszüge können durch Extraktion oder Destillation gewonnen werden. Für die Extraktion eignen sich die üblicherweise hierfür eingesetzten Lösungsmittel (Alkohole, Ketone und Ether), wobei die Verwendung von Wasser oder Wasser/Alkohol-Gemischen im Sinne der Erfindung bevorzugt ist. Der Gerbstoffgehalt nimmt mit der Extraktionsdauer zu. Es lösen sich dann vorwiegend auch hochmolekulare oder polymere Catechin- und Tannin-Gerbstoffe, die den Extrakten die charakteristische Dunkelfärbung verleihen. Teeauszüge, die durch Destillation gewonnen wurden, sind dagegen nahezu farblos und eignen sich besonders zur Herstellung farbneutraler und lagerungsstabiler Kosmetika. Der Wirkstoffgehalt in den Destillaten ist generell sehr viel niedriger als in den Extrakten.

Eine bevorzugte Ausführungsform der Zubereitung ist dadurch gekennzeichnet, daß als Auszug der Teepflanze das Wasserdampfdestillat von Blättern grünen, unfermentierten Tees von Camellia Sinensis in einer Menge von 1,0 - 20,0 Gew.-% in der Zubereitung enthalten ist. Besonders bevorzugt sind Mengen von 1,0 - 5,0 Gew.-%. Diese Wasserdampfdestillate sind farblos und enthalten neben den flüchtigen etherischen Ölen Theaflavin, wobei der Wirkstoffgehalt üblicherweise zwischen 0,01 - 0,5 Gew.-% liegt. Das antioxidative Potential liegt mit 0,00014 Vitamin-E-Einheiten im sehr niedrigen Bereich (Tabelle 1).

Nur in Kombination mit α-Tocopherylacetat, Tocopherol und Propylgallat beobachtet man eine unerwartet hohe Verstärkung der antioxidativen Wirkung, also einen Synergismus. Derartige Mischungen sind daher besonders gut als Antioxidantien-Kombination für Hautpflegemittel geeignet.

Die Antioxidantien-Kombination ist in den für kosmetische Rezepturen üblichen, physiologisch verträglichen Trägern enthalten. Neben Wasser und physiologisch geeigneten Lösungsmitteln können u.a. die üblichen pflegende Bestandteile, Öle, Wachse, Fette, rückfettende Substanzen, Verdickungsmittel, Emulgatoren, als Sonnenschutzfilter geeignete Substanzen und Duftstoffe enthalten sein. Die Zusammensetzung kann je nach Anforderung als wäßrige oder alkoholische Lösung, als Gel, Öl, W/O- oder O/W-Emulsion oder als Aerosol formuliert werden. Für geeignete Formulierungs-Grundlagen sei an dieser Stelle auf die in der Kosmetik üblichen Rezepturen verwiesen (K. Schrader, *Grundlagen und Rezepturen der Kosmetika*, 2. Aufl., Hüthig Buch Verlag, Heidelberg 1989, Seite 424-437, 442-451, 456-465).

Ebenfalls Gegenstand der Erfindung ist die Verwendung der erfindungsgemäßen Zubereitung zur pflegenden Behandlung der Haut. Die Antioxidantien-Kombination kann den Folgen des oxidativen Stresses vorbeugen und Regenerationsprozesse der Haut günstig beeinflussen. Die Herstellung der nachfolgend beschriebenen Emulsionen erfolgt nach in der Kosmetik allgemein üblichen Verfahren.

### Messung des antioxidativen Potentials (AOP)

Das antioxidative Potential (AOP) charakterisiert die "radikalfangenden" Eigenschaften einer Substanz oder eines Substanzgemisches.

Eine detaillierte Beschreibung der Methode und Apparatur findet sich in **DD 249 618 A3.** Die Methode beruht auf der durch Sauerstoffradikale induzierten Photochemilumineszenz von Luminol (3-Aminophthalsäurehydrazid) in Abwesenheit und in Gegenwart bestimmter Radikalfänger (Antioxidantien). Die Photochemilumineszenz in Abwesenheit von Antioxidantien dient als Leerwert. In Gegenwart von Radikalfängern wird eine Abnahme der Chemilumineszenz des Luminols beobachtet. Zur Kalibrierung des Systems wird eine definierte Menge Vitamin E zugegeben und die Chemilumineszenz in Gegenwart dieses Radikalfängers bestimmt. Dieser Wert dient als Referenz und wird als **1 Vitamin-E-Einheit** bezeichnet. Die durch die Prüfsubstanz hervorgerufene Lumineszenzabnahme wird in Relation zur Lumineszenzabnahme durch Vitamin E gesetzt und in Vitamin-E-Einheiten ausgedrückt. In Relation zu Vitamin E läßt sich somit das antioxidative Potential zahlreicher Substanzen und Substanzgemische bestimmen.

### Durchführung der AOP-Messungen

### 1. Prinzipieller Aufbau des Meßsystems

Die Apparatur besteht prinzipiell aus 2 Komponenten, einem Radikal-Generator und einem Detektorsystem.

Mittels einer UV-Strahlungsquelle werden im wäßrigen Medium durch Reduktion oder Anregung von Sauerstoff-Molekülen Superoxidradikal-Anionen oder Singulettsauerstoff erzeugt. Die entstandenen freien Radikale reagieren mit Luminol, dessen Chemilumineszenz über das Detektorsystem quantitativ erfaßt wird.

### 2. Benötigte Chemikalien

- Luminol, HPLC-grade (Fa. Baker)
- Methanol, HPLC-grade (Fa. Baker)
- Ethanol, HPLC-grade (Fa. Baker)
- Na₂CO₃
- Na₂EDTA·2H₂O
- NaOH
- α-Tocopherol (Fa. Merck)

### 3. Benötigte Geräte

Photochem, Fa. FAT, Berlin
Software: Poplab 2.0
Vibrofix

Übliches Labormaterial:
Spritzenfilter, 0,2 µm, Fa. Millipore

### 4.Versuchsdurchführung

### 4.1 Stammlösungen

### (A) ACL-Puffer-Stammlösung (ACL = Antioxidative Capacity of Lipid soluble substances)

Zur Herstellung des ACL-Puffers (pH = 11) wurden 10,6 g Na₂CO₃ (M=106,0 g/mol) und 0,0372 g Na₂EDTA·2H₂O (M=372,24 g/mol) eingewogen und mit Milli-Q-Wasser auf 1 Liter aufgefüllt. Die Pufferlösung wurde über ein 0,45 µm Spritzenfilter filtriert.

### (B) Methanolischer ACL - Puffer

Es wurden 50 mL der ACL-Pufferstammlösung (A) mit 950 mL Methanol (HPLC-grade) versetzt und solange gerührt, bis eine klare Lösung resultierte.

### (C) Luminol-Lösung

Zur Detektion wurde eine 1 mM Luminol-Lösung in 0,1 M Natronlauge verwendet. Die Lösung muß lichtgeschützt und gekühlt aufbewahrt werden.

### 4.2 Meßlösungen

### (D) Lösung zur Leerwertbestimmung

In ein verschließbares Autosamplergläschen (4 mL) wurden 2,0 mL des vorbereiteten klaren methanolischen ACL-Puffers pipettiert. Hierzu wurden ca. 100 µL der Luminollösung und soviel wasserfreies unvergälltes Ethanol gegeben, daß die Summe der Volumina von Ethanol und Luminollösung 0,5 mL ergab. Die Lösung wurde am Vibrofix wenige Sekunden lang homogenisiert und unverzüglich am Photochem vermessen (vgl. 4.4).

### (E) Proben-Lösung

In einem 10 mL-Meßkolben wurde die analytisch genau eingewogene Menge (ca. 0,5 g) der zu analysierenden Probe mit Methanol bis zur Marke aufgefüllt. Die Lösung wurde 10 min mit Ultraschall behandelt und anschließend filtriert.

### (F) Meßlösung

In ein verschließbares 4 mL-Autosamplergläschen wurden 2,0 mL methanolischen ACL-Puffer (vgl. B) pipettiert. Hierzu gab man dasselbe Volumen Luminollösung, das auch für die Leerwertbestimmung verwendet wurde, eine definierte Menge der filtrierten Probenlösung (E) und soviel wasserfreies unvergälltes Ethanol, daß die Summe der Volumina von Luminollösung, Probenlösung und Ethanol 0,5 mL ergab. Die Lösung wurde am Vibrofix wenige Sekunden lang homogenisiert und unverzüglich am Photochem vermessen.

### 4.3 Kalibrierung

Die Kalibierung erfolgte mit einem Vitamin-E-Standard. In einem 50 mL Meßkolben wurde eine analytisch genau eingewoge Menge d,l-α-Tocopherol (80 ± 5 mg) mit Methanol bis zur Marke aufgefüllt. Diese Lösung wurde mit Ethanol so verdünnt, daß sich ein Gehalt von 12 mg/L ergab.

Zur Kalibrierung wurden Lösungen (je 2,5 mL) mit jeweils 30, 50 und 100 µL Vitamin-E-Standardlösung hergestellt. Hierzu kombinierte man je 2 mL methanolische ACL-Pufferlösung mit demgleichen Volumen Luminollösung, das auch für die Leerwertbestimmung verwendet wurde, und gab jeweils 30, 50 und 100 µL Vitamin-E-Standardlösung und soviel wasserfreies unvergälltes Ethanol zu, daß die Summe der Volumina von Luminollösung, Vitamin-E-Standardlösung und Ethanol 0,5 mL ergab. Die Messung erfolgte wie unter 4.4. beschrieben.

### 4.4 Messung der Chemolumineszenz

Nach Einschalten des Gerätes und nach jeder Pause wurde die Chemilumineszenz der Leerwert-Lösung (vgl. D) bestimmt. Wenn die Meßwerte stabil blieben, konnte die eigentliche Messung gestartet werden. Die Meßdauer betrug pro Probe 180 Sekunden. Der Leerwert (Integral unter der Kurve) wurde von der Software automatisch bei den nachfolgenden Meßungen berücksichtigt.
Anschließend wurde das System auf Vitamin-E-Standards (vgl. 4.3) kalibriert, d.h. die Chemilumineszenz der drei Vitamin-E-Standards bestimmt. Dann wurde die Chemilumineszenz der Meßlösungen (F) der zu untersuchenden Proben bestimmt. Die Inhibition der Chemilumineszenz ergab sich aus dem Integral des Leerwertes minus dem Integral der Meßlösung normiert auf das Integral des Leerwertes.

### 5. Berechnung des Analysenergebnisses

Die für die Meßlösungen ermittelten Kurven wurden in Relation zu den Kalibrationskurven für Vitamin E gesetzt. Die für eine Substanzprobe spezifische Inhibition der Chemilumineszenz wurde in Vitamin-E-Äquivalente pro g Probe ausgedrückt (mg Vit. E /g Probe) und als sogenanntes *Antioxidatives Potential* (AOP) bezeichnet.

### Testergebnisse

Es wurden zahlreiche Kombinationen von Antioxidantien (Reinstoffe sowie Pflanzenextrakte) bezüglich ihres antioxidativen Potentials, insbesondere bezüglich synergistischer Effekte untersucht. Als Synergismus bezeichnet man das Verhalten, daß sich das antioxidative Potential einer Mischung nicht additiv aus dem AOP der Einzelkomponenten ergibt, sondern um ein Vielfaches verstärkt wird. Synergistische Effekte wurden für folgende Wirkstoffkombinationen (a + b + c) festgestellt:
a) Tocopherol und/oder Tocopherolester
b) Gallussäureester
c) Wasserdampfdestillat von grünem Tee

Exemplarisch sind in Tabelle 1 die Ergebnisse der Wirkstoffkombination (5) zusammengefaßt, die in den erfindungsgemäßen Formulierungsbeispielen enthalten ist und einen besonders ausgeprägten Synergismus, also einen "Booster-Effekt", aufweist.

**Tabelle 1**

| ***Substanzen*** | | ***AOP (Vitamin E-Einheiten)*** |
|---|---|---|
| (1) | Vitamin-E-Acetat | 1,0 |
| (2) | Controx® KS | 1,6 |
| (3) | Grüner Tee, Destillat | 0,00014 |
| (4) | Danox® 200 | 6,8 |
| | | |
| (5) | Mischung aus (1) + (2) + (3) + (4) | 97,0 |

### Bewertung:

Während das Destillat des grünen Tees ein sehr niedriges und das Vitamin-E-haltige Controx® KS und Vitamin-E-Acetat eine vergleichbare antioxidative Wirkung aufweisen, zeigt Propylgallat (Danox® 200) bereits ein deutlich höheres AOP (6,8 Vitamin-E-Einheiten). Jedoch erst die Wirkstoffkombination aus Vitamin-E-Acetat, Controx® KS, dem Destillat des grünen Tees und Danox® 200 liefert einen Booster-Effekt und führt zu einer Vervielfachung des AOP-Wertes (AOP = 97 Vitamin-E-Einheiten).

### Erfindungsgemäße Formulierungsbeispiele

Alle nachfolgenden Mengenangaben sind Gew.-% der handelsüblichen Substanzen oder Substanzgemische bezogen auf die Gesamtmenge der Zusammensetzung.

### Beispiel 1 (erfindungsgemäß):

Die Rezeptur enthält das erfindungsgemäße Antioxidantien-Gemisch.

| **Substanz** | **Menge in Gew.-%** |
|---|---|
| Montanov® 68 | 5,00 |
| Myritol® 318 | 5,50 |
| Lanette® 22 | 4,25 |
| Cutina® MD-V | 1,25 |
| Cetiol® V | 2,00 |
| Baysilon® M 350 | 0,50 |
| Vitamin-E-acetat | 0,50 |
| Controx® KS | 0,25 |
| Generol® 122 NE10D | 0,50 |
| UV-B-Filter | 3,00 |
| UV-A-Filter | 2,00 |
| Konservierungsmittel | 0,20 |
| | |
| Polyacrylat | 0,40 |
| Talkum | 0,80 |
| | |
| Grüner Tee, Destillat | 3,00 |
| Parfüm | 0,40 |
| Glycerin | 4,50 |
| Danox® 200 | 0,05 |
| NaOH, 10 %-ig | 0,46 |
| Wasser, demineralisiert | ad 100 |

### Beispiel 2 (erfindungsgemäß):

Die Rezeptur enthält das erfindungsgemäße Antioxidantien-Gemisch.

| **Substanz** | **Menge in Gew.-%** |
|---|---|
| Lipoid® S75-3 | 1,5 |
| Stenol® 1618 | 2,0 |
| Cetiol® SB 45 | 1,0 |
| Cegesoft® C24 | 6,0 |
| Prisorine® IPIS | 5,0 |
| Controx® KS | 0,25 |
| Tocopherolacetat | 0,5 |
| UV-A-Filter | 1,0 |
| UV-B-Filter | 4,0 |
| Konservierungsmittel | 0,4 |
| | |
| Polyacrylat | 0,50 |
| Methocel® E4M (prem.) | 0,1 |
| | |
| Grüner Tee, Destillat | 3,0 |
| Danox® 200 | 0,05 |
| Parfum | 0,2 |
| | |
| 1,2-Propylenglykol | 5,0 |
| Glycerin | 5,0 |
| NaOH (10%-ig) | 1,25 |
| Wasser, dest. | ad 100 |

### Anhang

### Verzeichnis der Warenzeichen und Handelsnamen der verwendeten Rohstoffe

1) Montanov® 68:
   INCI: Cetearyl alcohol, Cetearyl glucoside
   Hersteller: Seppic (Interorgana)
2) Myritol® 318
   INCI: Caprylic/Capric triglyceride
   Hersteller: Henkel KGaA
3) Lanette® 22
   INCI: Behenyl alcohol
   Hersteller: Henkel (Sidobre-Sinnova)
4) Cutina® MD-V
   INCI: Stearyl stearate
   Hersteller: Henkel KGaA
5) Cetiol® V
   INCI: Decyl oleate
   Hersteller: Henkel KGaA
6) Baysilon® M 350
   INCI: Dimethicone
   Hersteller: Bayer AG
7) Vitamin-E-Acetat
   INCI: Tocopheryl acetate
   Hersteller: BASF
8) Controx® KS
   INCI: Tocopherol, hydrogenated palm glycerides citrate
   Tocopherolgehalt: ca. 56 Gew.-%
   Hersteller: Henkel (Grünau)
9) Generol® 122 NE10D
   INCI: PEG-10 soya sterol
   Hersteller: Grünau (Henkel KGaA)
10) Herbasol®-Destillat Tee Grün Spezial Henkel, ohne Alkohol
   INCI: Aqua, camellia sinensis extract, benzoic acid, polysorbate-20
   Wasserdampfdestillat: mit 0.75 Gew% Benzoesäure, ca. 2 Gew.-% Polysorbat 20 100 kg Destillat enthalten die flüchtigen Bestandteile von 100 kg grünem Tee Wirkstoffgehalt: ca. 0.01 - 0.5 Gew.-%
   Hersteller: Cosmetochem AG, Steinhausen, Schweiz
11) Danox® 200
   INCI: Propylgallat
   Hersteller: IFSC (Rahn)
12) Lipoid® S75-3
   INCI: Hydrogenated lecithin
   Hersteller: Lipoid GmbH
13)Stenol® 1618
   INCI: Cetearyl alcohol
   Hersteller: Henkel KGaA
14) Cetiol® SB 45
   INCI: Shea Butter
      Butyrospermum Parkii
   Hersteller: Henkel KGaA
15) Cegesoft® C24
   INCI: Octyl palmitate
   Hersteller: Henkel (Grünau)
16) Prisorine® IPIS 2021
   INCI: Isopropyl isostearate
   Hersteller: Unichema
17) Methocel® E4M premium
   INCI: Hydroxypropyl methylcellulose
   Hersteller: Dow Chemical (Colorcon Ltd.)

## Patentansprüche

1. Kosmetische Zubereitung, **dadurch gekennzeichnet, daß** als Antioxidans eine Kombination aus
(a) mindestens einem Tocopherol und/oder Tocopherol-Ester
(b) mindestens einem Gallussäure-Ester und
(c) einem Auszug der Tee-Pflanze
in einem zur topischen Applikation auf der Haut geeigneten Träger enthalten ist.

2. Zubereitung gemäß Anspruch 1, **dadurch gekennzeichnet, daß** als Komponente (a) ein natürliches oder synthetisches α-Tocopherol in einer Menge von 0,005- 2,0 Gew.-% enthalten ist.

3. Zubereitung gemäß Anspruch 2, **dadurch gekennzeichnet, daß** zusätzlich α-Tocopherylacetat in einer Menge von 0,005 - 5,0 Gew.-% enthalten ist.

4. Zubereitung gemäß einem der Ansprüche 1 bis 3, **dadurch gekennzeichnet, daß** als Gallussäureester Propylgallat in einer Menge von 0,005 - 2,0 Gew.-% enthalten ist.

5. Zubereitung gemäß einem der Ansprüche 1 bis 4, **dadurch gekennzeichnet, daß** als Auszug der Teepflanze das Wasserdampfdestillat von Blättern grünen, unfermentierten Tees von Camellia Sinensis in einer Menge von 1,0 - 20,0 Gew.-% in der Zubereitung enthalten ist.

6. Nicht therapeutische Verwendung einer Zubereitung nach einem der Ansprüche 1 bis 5 zur pflegenden Behandlung der Haut.

7. Nicht therapeutische Verwendung einer Zubereitung nach einem der Ansprüche 1 bis 5 zur vorbeugenden Behandlung der Hautalterung.

8. Nicht therapeutische Verwendung einer Zubereitung nach einem der Ansprüche 1 bis 5 zur Verbesserung des Erscheinungsbildes der Altershaut.

## Claims

1. A cosmetic preparation, **characterized in that** it contains as antioxidant a combination of
(a) at least one tocopherol and/or tocopherol ester,
(b) at least one gallic acid ester and
(c) an extract of the tea plant
in a carrier suitable for topical application to the skin.

2. A preparation as claimed in claim 1, **characterized in that** a natural or synthetic α-tocopherol in a quantity of 0.005 to 2.0% by weight is present as component (a).

3. A preparation as claimed in claim 2, **characterized in that** α-tocopheryl acetate is additionally present in a quantity of 0.005 to 5.0% by weight.

4. A preparation as claimed in any of claims 1 to 3, **characterized in that** propyl gallate is present as the gallic acid ester in a quantity of 0.005 to 2.0% by weight.

5. A preparation as claimed in any of claims 1 to 4, **characterized in that** the steam distillate of leaves of green unfermented tea of Camellia sinensis is present in the preparation as the tea plant extract in a quantity of 1.0 to 20.0% by weight

6. The non-therapeutic use of the preparation claimed in any of claims 1 to 5 for skin care.

7. The non-therapeutic use of the preparation claimed in any of claims 1 to 5 for the preventive treatment of aging of the skin.

8. The non-therapeutic use of the preparation claimed in any of claims 1 to 5 for improving the appearance of aged skin.

## Revendications

1. Préparation cosmétique,
**caractérisée en ce que**
comme agent anti-oxydant une combinaison à base de
a) au moins un tocophérol et/ou un ester de tocophérol
b) au moins un ester d'acide gallique
c) un extrait de la plante de thé
est présente dans un vecteur approprié pour l'application topique sur la peau.

2. Préparation conformément à la revendication 1,
**caractérisée en ce que**
comme composant (a) un α-tocophérol naturel ou synthétique, est présent en une quantité allant de 0,005 à 2 % en poids.

3. Préparation conformément à la revendication ou 2,
**caractérisée en ce que**
en supplément de l'acétate d'α-tocophérol est présent en une quantité de 0,005 à 5,0 % en poids.

4. Préparation conformément à l'une des revendications 1 à 3,
**caractérisée en ce que** comme ester d'acide gallique, du gallate de propyle est présent en une quantité variante de 0,005 à 2 % en poids.

5. Préparation conformément à l'une quelconque des revendications 1 à 4,
**caractérisée en ce qu'**
elle contient comme extrait de plante de thé le distillat sous vapeur d'eau de feuilles de thé vert, non fermenté de Camellia Sinensis, en quantité de 1,0 à 20,0 % en poids.

6. Utilisation non thérapeutique d'une préparation selon l'une des revendications 1 à 5,
pour le traitement soignant de la peau.

7. Utilisation non thérapeutique d'une préparation selon l'une des revendications 1 à 5,
pour le traitement préventif du vieillissement de la peau.

8. Utilisation non thérapeutique d'une préparation selon l'une des revendications 1 à 5,
en vue d'améliorer l'aspect de la peau âgée.
